# EUROPÄISCHE PATENTSCHRIFT

(11) **EP 0 855 172 B1**
(45) Veröffentlichungstag und Bekanntmachung des Hinweises auf die Patenterteilung: **08.01.2003**
(21) Anmeldenummer: 98100245.4
(22) Anmeldetag: 09.01.1998
(51) Int. Cl.: A61F 2/38

(54) **Fixation eines Tibiateils auf einem Tibiaplateau einer Kniegelenkendoprothese**
Fastening of a tibial piece on a tibilal tray of a knee prosthesis
Fixation d'une pièce tibiale sur le plateau tibial d'une prothèse de genou

(30) Priorität: 17.01.1997 DE 19701622; 01.03.1997 DE 19708375
(43) Veröffentlichungstag der Anmeldung: 29.07.1998
(73) Patentinhaber: CeramTec AG Innovative Ceramic Engineering, 73207 Plochingen (DE)
(72) Erfinder: Bädorf, Dirk, 50226 Frechen (DE); Pfaff, Hans-Georg, 73760 Ostfildern (DE)
(74) Vertreter: Scherzberg, Andreas, Dr.

(56) Entgegenhaltungen:
- EP-A- 0 306 744
- EP-A- 0 672 397
- US-A- 3 958 278
- US-A- 4 944 757
- US-A- 5 358 527
- US-A- 5 480 446

## Beschreibung

Die Erfindung betrifft eine Kniegelenkendoprothese.

Aus der EP 0 442 330 B1 ist eine Kniegelenkendoprothese mit einem Femurteil, einem Meniskuselement und einem Tibiateil bekannt, wobei das Femurteil und das Tibiateil mit Lagerflächen an zugeordneten Lagerflächen des Meniskuselements anliegen.

Das Tibiateil ist mit einem Tibiaplateau fest verbunden, wobei das Tibiaplateau im tibialen Knochen verankert ist und mit diesem verwachsen soll. Femurteil, Tibiateil und Meniskuselement sind bevorzugt aus einem keramischen Werkstoff hergestellt.

US-A-5 480 446 zeigt eine Verbindung zwischen Tibiaplateau und Tibiateil.

Der Erfindung liegt die Aufgabe zugrunde, eine mechanisch stabile Verankerung des Tibiateils auf dem Tibiaplateau zu schaffen, die gegebenenfalls auch am implantierten Gelenk nachträglich gelöst und damit das Tibiateil ausgetauscht werden kann.

Erfindungsgemäß wird diese Aufgabe dadurch gelöst, daß das oder die Tibiateile mit dem Tibiaplateau lösbar verklemmt, verschraubt und/oder verspannt sind, und auf dem Tibiaplateau ein Spannelement über eine Fixierschraube befestigt ist, welches die Tibiateile verklemmt und/oder verspannt. Durch Lösen der Fixierschraube läßt sich das Spannelement entfernen und so die Tibiateile entnehmen.

Hierdurch sind die Tibiateile zwar fest auf dem Tibiaplateau verankert, jedoch ist die Verankerung gegebenenfallls auch am implantierten Gelenk nachträglich lösbar, wodurch das Tibiateil oder die Tibiateile leicht ausgetauscht werden können.

Das Spannelement ist zwischen den Tibiateilen angeordnet und verankert gleichzeitig beide Tibiateile auf dem Tibiaplateau.

Die zum Spannelement gewandte Seitenfläche des Tibiateils und die angrenzende Seitenfläche des Spannelements weisen eine zueinander passende Abschrägung auf, wobei die Abschrägung des Spannelements auf der Abschrägung des Tibiateils aufliegt. Hierdurch ist eine stabile Verankerung durch Klemmen bzw. Verankern erreicht.

In vorteilhafter Ausgestaltung sind die Tibiateile in eine Einbuchtung des Tibiaplateaus eingelegt. Dies vereinfacht die Verankerung, da die Tibiateile durch den Rand der Einbuchtung horizontal fixiert sind.

Vorteilhafterweise ist zumindest eine der Seitenflächen der Einbuchtung mit einer Einkerbung versehen, die auf dem Tibiateil aufliegt. Das Tibiateil weist hierzu eine an die Einkerbung angepaßte Schräge auf, die in die Einkerbung hineinreicht.

In bevorzugter Ausführungsform bestehen die Tibiateile aus einem keramischen Werkstoff, wie z. B. Aluminiumoxid, während das Tibiaplateau aus z. B. einer Titanlegierung besteht.

Weitere Merkmale der Erfindung ergeben sich aus den beiden Figuren, die nachfolgend beschrieben sind.

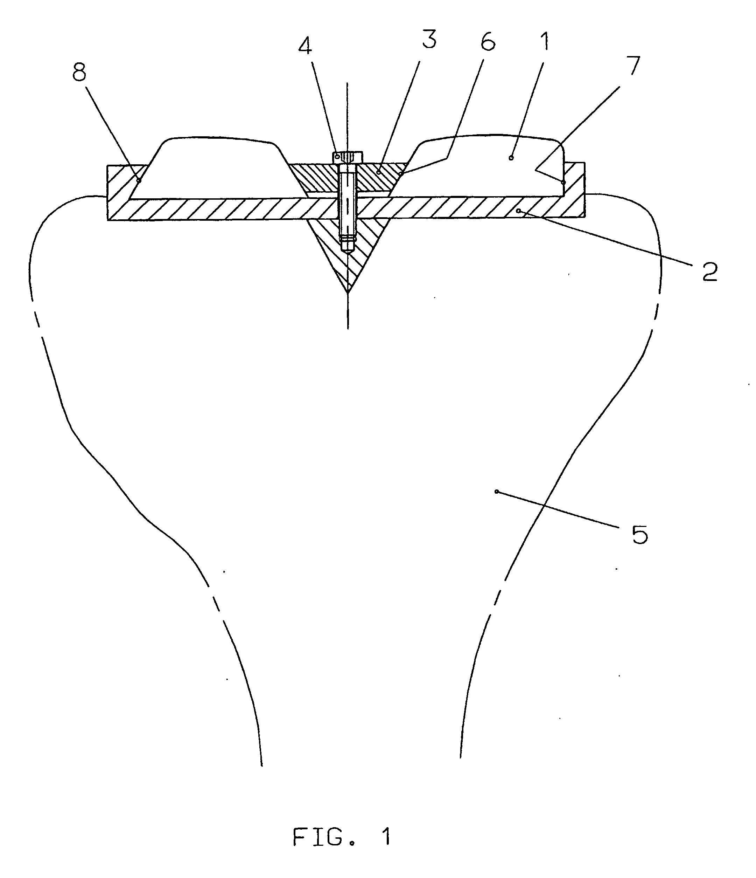

Fig. 1 zeigt einen Schnitt durch einen tibialen Knochen 5 mit aufgesetztem Tibiaplateau 2 und eingesetzten Tibiateilen 1. Nicht gezeigt ist der Rest der Kniegelenkendoprothese, nämlich das Femurteil und die Meniskuselemente.

Das Tibiaplateau 2 ist plattenförmig ausgebildet und weist eine Einbuchtung 7 auf der zum Femurteil gewandten Seite auf. In diese Einbuchtung 7 sind zwei Tibiateile 1 aus einem keramischen Werkstoff eingesetzt. Diese Tibiateile 1 berühren die Seitenflächen der Einbuchtung 7 und sind über ein mittig angeordnetes Spannelement 3 verankert. Das Spannelement 3 ist über eine Fixierschraube 4 auf dem Tibiaplateau 2 befestigt. Das Spannelement 3 verklemmt und/oder verspannt gleichzeitig beide Tibiateile 1.

Zur besseren Verankerung sind die aneinanderliegenden Seitenflächen des Tibiateils 1 und des Spannelements 3 mit einer zueinanderpassenden Abschrägung 6 versehen, wobei die Abschrägung des Spannelements 3 auf der Abschrägung des Tibiateils 1 aufliegt.

Zur zusätzlichen Verankerung ist mindestens eine der Seitenflächen der Einbuchtung 7 mit einer Einkerbung 8 versehen, in die die angrenzende Seitenfläche des Tibiateils 1 hineinragt, so daß die Seitenfläche der Einkerbung 8 auf der Schräge des Tibiateils 1 aufliegt. Diese Einkerbung 8 ist vorteilhafterweise auf allen Seitenflächen des Tibiaplateaus 2 angeordnet.

Die Tibiateile 1 bestehen aus einem keramischen Werkstoff, während das Spannelement 3 wie auch das Tibiaplateau 2 aus einer Titanlegierung hergestellt sind.

Durch Lösen der Fixierschraube 4 und Entfernen des Spannelements 3 läßt sich das Tibiateil 1 bzw. die beiden Tibiateile 1 leicht aus dem Tibiaplateau 2 nach medial entnehmen.

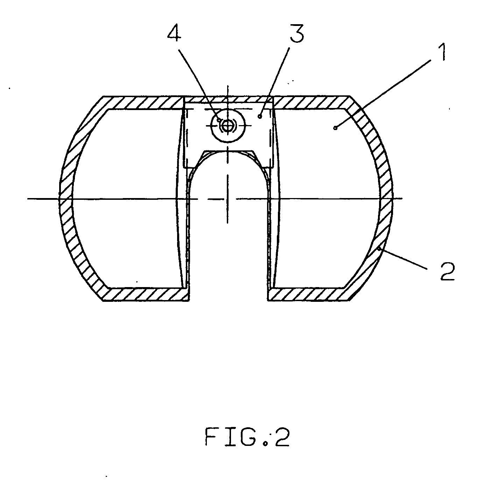

Fig. 2 zeigt eine Draufsicht auf ein Tibiaplateau 2 mit eingesetzten Tibiateilen 1 gemäß der Fig. 1 von der Femurseite. Gut zu erkennen ist, wie das Spannelement 3 auf der Abschrägung 6 (siehe Fig. 1) der Tibiateile 1 aufliegt und diese so festlegt.

Die Tibiateile 1 können auch direkt mit dem Tibiaplateau 2 verschraubt sein. Hierzu ist eine Bohrung in den Tibiateilen 1 erforderlich, in der eine Schraube eingelassen ist. Die Befestigung kann auf alle erdenklichen Arten erfolgen, sie muß jedoch lösbar ausgebildet sein.

## Patentansprüche

1. Kniegelenkendoprothese mit einem im Femur verankerten Femurteil und einem mit dem tibialen Knochen verbundenen Tibiaplateau (2) auf dem als Gleitflächen Tibiateile (1) angeordnet sind und vorzugsweise mit zwischen dem Femurteil und den Tibiateilen (1) angeordneten Meniskuselementen, und auf dem Tibiaplateau (2) ein Spannelement (3) über eine Fixierschraube (4) befestigt ist, welches die Tibiateile (1) verklemmt und/oder verspannt, und das Spannelement (3) zwischen den Tibiateilen (1) angeordnet ist und gleichzeitig beide Tibiateile (1) auf dem Tibiaplateau (2) verankert; wobei die zum Spannelement (3) gewandte Seitenfläche des Tibiateils (1) und die angrenzende Seitenfläche des Spannelements (3) eine zueinanderpassende Abschrägung aufweisen und die Abschrägung des Spannelements (3) auf der Abschrägung des Tibiateils (1) aufliegt.

2. Kniegelenkendoprothese nach Anspruch 1, **dadurch gekennzeichnet, dass** die Tibiateile (1) in eine Einbuchtung (7) des Tibiaplateaus (2) eingelegt sind.

3. Kniegelenkendoprothese nach Anspruch 2, **dadurch gekennzeichnet, dass** zumindest eine der Seitenflächen der Einbuchtung (7) mit einer Einkerbung (8) versehen ist, die auf dem Tibiateil (1) aufliegt.

4. Kniegelenkendoprothese nach einem der Ansprüche 1 bis 3, **dadurch gekennzeichnet, dass** die Tibiateile (1) aus einem keramischen Werkstoff bestehen.

## Claims

1. Knee joint endoprosthesis which has a femur portion which is anchored in the femur and a tibia plateau (2) which is connected to the tibial bone and on which tibia portions (1) are arranged as slide faces, the endoprosthesis preferably having meniscus elements arranged between the femur portion and the tibia portions (1) there being secured on the tibia plateau (2), by way of a fixing screw (4), a tensioning element (3) which clamps and/or tensions the tibia portions (1), the tensioning element (3) being arranged between the tibia portions (1) and simultaneously anchoring both tibia portions (1) on the tibia plateau (2), with the side face of the tibia portion (1) that faces the tensioning element (3) and the adjacent side face of the tensioning element (3) having matching bevels and the bevel of the tensioning element (3) resting on the bevel of the tibia portion (1).

2. Knee joint endoprosthesis according to claim 1, **characterised in that** the tibia portions (1) are inserted into a recess (7) of the tibia plateau (2).

3. Knee joint endoprosthesis according to claim 2, **characterised in that** at least one of the side faces of the recess (7) is provided with an indentation (8) which rests on the tibia portion (1).

4. Knee joint endoprosthesis according to one of claims 1 to 3, **characterised in that** the tibia portions (1) are made from a ceramic material.

## Revendications

1. Endoprothèse du genou, avec une pièce fémorale ancrée dans le fémur et un plateau tibial (2) lié à l'os tibial sur lequel sont disposées des pièces tibiales (1) en tant que surfaces de glissement, avec de préférence des éléments de ménisque disposés entre la pièce fémorale et les pièces tibiales (1), avec sur le plateau tibial (2) un élément de bridage (3) qui est fixé à l'aide d'une vis de fixation (4) et qui bloque et/ou bride les pièces tibiales (1), l'élément de bridage (3) étant disposé entre les pièces tibiales (1) et bloquant simultanément les deux pièces tibiales (1) sur le plateau tibial (2), la face latérale de la pièce tibiale (1) tournée vers l'élément de bridage (3) et la face latérale contiguë de l'élément de bridage (3) présentant des surfaces inclinées mutuellement adaptées et la surface inclinée de l'élément de bridage (3) étant en appui sur la surface inclinée de la pièce tibiale (1).

2. Endoprothèse du genou selon la revendication 1, **caractérisée en ce que** les pièces tibiales (1) sont insérées dans une échancrure (7) du plateau tibial (2).

3. Endoprothèse du genou selon la revendication 1, **caractérisée en ce qu'**au moins une des surfaces latérales de l'échancrure (7) est pourvue d'une contre-dépouille (8) qui est en contact avec la pièce tibiale (1).

4. Endoprothèse du genou selon une des revendications 1 à 3, **caractérisée en ce que** les pièces tibiales (1) sont en un matériau céramique.
